(19) Europäisches Patentamt
European Patent Office
Office européen des brevets

(11) Numéro de publication: **0 399 885 A1**

(12) **DEMANDE DE BREVET EUROPEEN**

(21) Numéro de dépôt: **90401338.0**

(51) Int. Cl.[5]: **C07K 5/08**

(22) Date de dépôt: **21.05.90**

(30) Priorité: **24.05.89 FR 8906777**

(43) Date de publication de la demande:
**28.11.90 Bulletin 90/48**

(84) Etats contractants désignés:
**AT BE CH DE DK ES FR GB GR IT LI LU NL SE**

(71) Demandeur: **RHONE-POULENC CHIMIE**
**25, quai Paul Doumer**
**F-92408 Courbevoie Cédex(FR)**

(72) Inventeur: **Bernard, Jean-Marie**
**Route du Large**
**Saint-Laurent d'Agny, F-69440 Mornant(FR)**

(74) Mandataire: **Ricalens, François et al**
**RHONE-POULENC CHIMIE Service Brevets**
**Chimie 25, quai Paul Doumer**
**F-92408 Courbevoie(FR)**

(54) **Nouveau tripeptide, son procédé de préparation et son utilisation.**

(57) La présente invention concerne un nouvel intermédiaire de synthèse peptidique ayant la composition suivante :

Leucyl - Arginyl - Proline ainsi que son procédé de préparation.

EP 0 399 885 A1

Xerox Copy Centre

## NOUVEAU TRIPEPTIDE, SON PROCEDE DE PREPARATION ET SON UTILISATION

La présente invention concerne un nouveau tripeptide, son procédé de préparation ainsi que son utilisation. Ce tripeptide est un intermédiaire de synthèse important pour notamment la préparation des hormones permettant la libération de l'hormone lutéinisante plus connue sous l'abréviation de LHRH.

La LHRH hormone hypothalamique a pour fonction la régulation de la sécrétion des hormones gonadotropes. En raison de sa demande au niveau clinique, de nombreux dérivés de cette dernière ont été synthétisés. La majorité de ces composés comporte à l'intérieur de leur molécule le tripeptide objet de la présente invention qui n'a à ce jour jamais été synthétisé.

Ce tripeptide qui est nouveau et fait l'objet de la présente demande répond à la formule suivante :

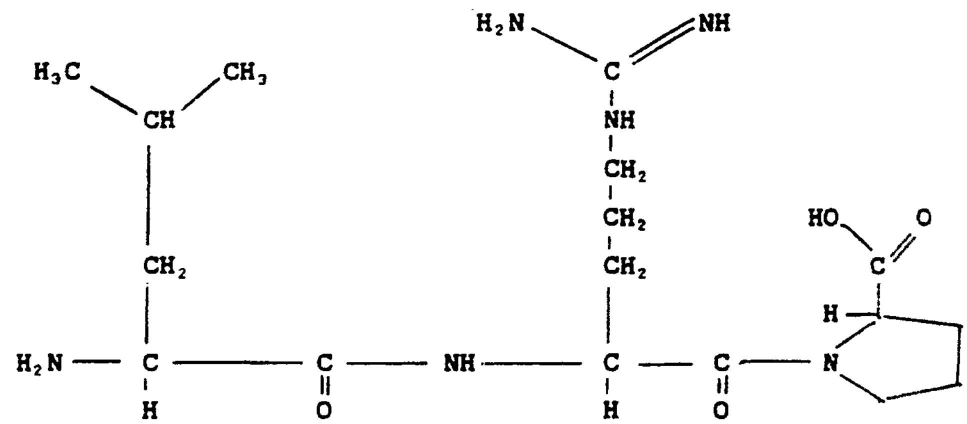

encore plus communément appelé Leucyl - Arginyl - Proline.

Il peut aussi être protégé sur au moins l'une de ses fonctions acide et/ou amine par un groupe protecteur classique utilisé en synthèse peptidique tel que notamment décrit par Gross et Meienhofer, (The peptides, vol.3, Academic Press, 1981).

Parmi les groupes protecteurs de la fonction amine on peut citer à titre d'exemples non limitatifs les groupes :

tertiobutyloxycarbonyle connu sous l'abréviation BOC
benzyloxycarbonyle connu sous l'abréviation Z
fluorénylméthyloxycarbonyle connu sous l'abréviation FMOC
benzoyle
trifluoroacétyle
uréthane
formyle
trityle
nitrophénylsulfényle

Parmi les groupes protecteurs de la fonction acide C-terminale on peut également citer à titre non limitatif

- les groupes esters et particulièrement les esters d'alkyle dont les chaînes alkyle contiennent 1 à 20 atomes de carbone et tout particulièrement les esters de méthyle, d'éthyle, de phényle, de benzyle ou de tertiobutyle.
- les groupes amides primaires ou substitués sur l'atome d'azote

Le tripeptide objet de la présente invention est préparé selon un procédé qui consiste

- dans une première étape à mettre en présence la L-nitroArginine protégée sur sa fonction amine et la L-Proline protégée sur sa fonction acide en présence d'un activateur et d'au moins une base dans un solvant organique.
- dans une deuxième étape à hydrogéner le dipeptide obtenu à la première étape de façon à éliminer le groupe protecteur de l'argine, le groupe protecteur de la proline et de façon à réduire le radical $NO_2$
- dans une troisième étape on met en contact le dipeptide issu de la deuxième étape avec la L-leucine protégée sur sa fonction amine en présence d'un activateur, d'au moins une base et d'un nucléophile auxiliaire.

Le groupe protecteur d'une des fonctions amine de la L-nitroarginine est de préférence un groupe benzyloxycarbonyle le groupe protecteur de la 2ème fonction amine étant un groupe nitro et le groupe protecteur de la fonction acide de la Proline est de préférence un groupe benzyle.

Le groupe protecteur de la fonction amine de la L-leucine est aussi de préférence un groupe benzyloxycarbonyle.

Au cours de la première étape, l'activateur de condensation peut être choisi parmi notamment les composés suivants :
les chlorures d'acide d'alkyles, d'aralkyles, d'aryles
les anhydrides d'acide phosphorés
les carbodiimides
les esters actives

On peut citer à titre d'exemple :
le chlorure de pivaloyle
le chlorure de dimethoxytriazine
le réactif de Castro (BOP)
le chloroformiate d'isobutyle
la dicyclohexylcarbodiimide (DCC)

On préfère utiliser le chlorure de pivaloyle.

L'utilisation par exemple du chlorure de diméthoxytriazine en léger excès par rapport à l'arginine provoque la formation d'un intermédiaire dimethoxytriazine-Proline protégé qui est perdu pour la suite de la réaction.

L'utilisation du dicyclohexylcarbodiimide provoque l'apparition d'un précipité important de dicyclohexylurée difficile à séparer.

Le rapport molaire de l'agent d'activation à l'argine protégée est de préférence compris entre 1 et 1,5. Le rapport molaire de la L-Proline à la L-nitroarginine est de préférence compris entre 1 et 2.

La base d'activation et de condensation est choisie parmi les bases suivantes, les bases azotées tertiaires, les bases alcalines et notamment parmi :
la N-méthylmorpholine
la triethylamine
le carbonate de sodium
la diisopropyléthylamine

Pour l'étape d'activation la base est choisie de préférence parmi les amines solubles dans les milieux organiques et ne présentant pas une basicité trop élevée pouvant provoquer une racémisation de l'arginine. On préfère ainsi utiliser des bases amines tertiaires encombrées telles que
la N-méthylmorpholine
la diisopropylethylamine
la N-méthylpipéridine

Le rapport molaire de ladite base à la L-nitroarginine est de préférence compris entre 1 et 2.

Pour l'étape de condensation il est nécessaire de déplacer le chlorhydrate de la proline protégée avec une base relativement forte ayant un PK supérieur à celui de la proline protégée. Il est donc préférable d'utiliser une base choisie parmi notamment :
la triéthylamine
le carbonate acide de sodium

Le rapport molaire de ladite base à la L-Proline est de préférence compris entre 1 et 2.

Le solvant utilisé lors de la première étape d'activation et de condensation est de préférence choisi parmi :
le dichlorométhane
l'acétate d'éthyle
le dioxane
le tétrahydrofuranne
le diméthylformamide
le tertiobutanol
le toluène
le diméthoxyéthane

On préfère utiliser le dichlorométhane.

La température réactionnelle pratiquée lors de l'étape d'activation et lors de l'étape de couplage est de préférence comprise entre - 30° C et 40° C et de préférence comprise entre - 15° C et la température ambiante.

A la fin de la première étape le dipeptide protégé Arginyl-Proline est lavé puis séparé par cristallisation dans l'acétate d'éthyle.

La deuxième étape consiste à éliminer les groupes protecteurs portés par l'Arginine et la Proline.

Cette double réaction, est réalisée par hydrogénation du dipeptide Arginyl-Proline en milieu acide.

L'hydrogénation de la deuxième étape est réalisée en présence d'un catalyseur à base d'un métal précieux déposé sur un support inerte et en présence d'un acide minéral ou organique.

Le métal précieux est de préférence le palladium déposé sur charbon.

On préfère utiliser une quantité pondérale de métal précieux par rapport au dipeptide comprise entre 0,5 et 5 % ce qui correspond à environ 5 à 50 % en poids de catalyseur supporté par rapport au poids de dipeptide.

L'hydrogénation est réalisée de préférence dans un solvant constitué d'un mélange acide carboxylique/eau contenant en plus au minimum 2 équivalents d'acide fort. Chaque équivalent d'acide fort permet de bloquer les fonctions guanidine et amine primaire sous forme de sels, ceci afin d'éviter la formation de dicetopipérazine.

L'acide est choisi parmi
l'acide chlorhydrique
l'acide bromhydrique
l'acide sulfurique
l'acide paratoluènesulfonique
l'acide méthanesulfonique

On préfère utiliser l'acide chlorhydrique.

Le solvant est de préférence constitué d'un mélange acide carboxylique/eau encore plus préférentiellement d'un mélange acide acétique/eau contenant tout particulièrement 90 % d'acide acétique en volume.

La pression d'hydrogène utilisée est avantageusement comprise entre 1 et 20 bar et de préférence entre 1 et 5 bar.

La température réactionnelle est avantageusement comprise entre 0° et 100° C et de préférence entre la température ambiante et 50° C.

Le dipeptide Arginyl-Proline non protégé obtenu à cette étape est purifié puis éventuellement séparé par cristallisation dans l'acétate d'éthyle.

La troisième étape du procédé selon l'invention consiste à condenser le dipeptide Arginyl-Proline sur la leucine.

Cette condensation se fait en 3 étapes :
une étape d'activation de la leucine
une étape de condensation sur le dipeptide
une étape de séparation

L'étape d'activation est réalisée par addition d'un agent d'activation choisi parmi le chloroformiate d'isobutyle ou le chlorure de pivaloyle.

Les autres réactifs d'activation étant soit trop onéreux (réactif de Castro) soit donnent de moins bons résultats (chlorure de dimethoxytriazine) soit entraînent la formation de produits secondaires difficilement séparables (dicychlohexylcarbodiimide).

Le chloroformiate d'isobutyle ou le chlorure de pivaloyle sont des produits peu onéreux, qui ne donnent que peu de produits secondaires éliminés facilement par cristallisation.

Le rapport molaire de l'agent d'activation à la L-leucine est avantageusement compris entre 0,5 et 1.

La formation de l'anhydride mixte (leucine-pivaloyl protégée ou leucine-isobutyl protégée) est favorisée par la présence d'une base faible d'activation choisie de préférence parmi les amines tertiaires encombrées telles que :
la N-methylmorpholine
la diisopropyl éthylamine

Le rapport molaire de la ditebase à la leucine est avantageusement compris entre 1 et 2.

Cet anhydride mixte est déplacé par l'utilisation d'un nucléophile auxiliaire qui a pour fonction d'éviter la formation d'un anhydride mixte entre la leucine protégée et le dipeptide Arginyl- Proline (Z Leu-Arg-Pro-CO-O-CO-Leu-Z) par attaque du carboxylate du di ou tripeptide Arg-Pro ou Leu-Arg-Pro sur la leucine activée et protégée.

Le nucléophile auxiliaire est choisi parmi par exemple l'un des réactifs suivants :
l'hydroxybenzotriazole
l'hydroxysuccinimide
le thiazolidine-thiol de formule

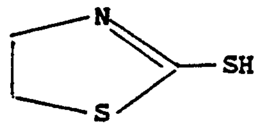

- l'hydroxy-3, dihydro-3,4, benzotriazil 1,2,3 one 4 de formule

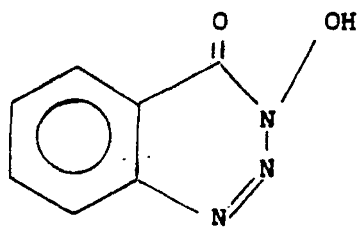

On préfère utiliser l'hydroxybenzotriazole.

Le rapport molaire du nucléophile auxiliaire de la leucine est avantageusement compris entre 0,1 et 2 et de préférence entre 0,8 et 1,2.

La réaction d'activation est réalisée dans les mêmes solvants et dans les mêmes conditions que celles décrites à la première étape du procédé selon l'invention.

On préfère utiliser le dichlorométhane ou le diméthylformamide et une température d'environ - 15° C.

L'étape de condensation est réalisée par introduction dans le milieu contenant la leucine activée du dipeptide en solution dans un solvant choisi parmi

le diméthylformamide
le dichlorométhane
le trifluoroéthanol
l'acide acétique
l'isopropanol
le tétrahydrofurane
l'acétonitrile
le diméthoxyèthane

On préfère utiliser un solvant constitué de diméthylformamide et d'eau.

La base utilisée lors de la condensation est choisie notamment parmi :

le carbonate acide de sodium
la triéthylamine
la tributylamine
l'acétate de sodium

Le rapport molaire de la dite base à la Leucine est avantageusement compris entre 1 et 4 et de préférence entre 1,5 et 2,5.

La température de condensation est avantageusement comprise entre - 30° et 50° C et de préférence entre 0° C et la température ambiante.

Le rapport molaire leucine activée/dipeptide est avantageusement compris entre 1,5 et 2.

Le tripeptide obtenu est extrait de préférence par un mélange acétate d'éthyle-butanol ou de façon plus générale par un mélange ester-alcool non miscible à l'eau. On concentre ensuite le tripeptide et on le précipite en reprenant le milieu à l'isopropanol et en le versant sur de l'éther isopropylique.

La présente invention sera plus complètement décrite à l'aide des exemples suivants qui ne doivent pas être considérés comme limitatifs de l'invention.

Exemple 1ère étape du procédé

Mode opératoire

A température ambiante, dans un tricol de 250 ml sont ajoutés successivement 19 g de Z-Arg ($NO_2$)-COOH, 95 ml de chlorure de méthylène et 5,82 ml de N-méthylmorpholine. La bouillie est agitée pendant

cinq minutes puis est refroidie à - 5° C.

6,5 ml de chlorure de pivaloyle sont ajoutés en dix minutes afin de maintenir la température constante à - 5° C.

La masse réactionnelle homogène est agitée pendant 2 heures à - 5° C, et prend une couleur orangée.

A cette masse réactionnelle sont ajoutés 15,8 g de Chlorhydrate de l'ester benzylique de la L-proline, en poudre.

Puis, la triéthylamine (8,8 ml) est ajoutée en 10 minutes de façon à maintenir la température du milieu réactionnel à - 5° C (la réaction est exothermique).

Après 2 heures de réaction à - 5° C, on porte le mélange réactionnel à 300 ml par addition de chlorure de méthylène et de 30 ml de diisopropyl éther et on procède à l'extraction.

La phase organique est lavée successivement par 100 ml d'eau, 100 ml de $KHCO_3$ 2,5 M, par 3 fois 50 ml de $KHCO_3$ 2,5 M, par 4 fois 50 ml de HClN et finalement par de l'eau, jusqu'à ce que le pH de la phase aqueuse soit égal à 5.

La solution organique est concentrée à 100 ml et 400 ml d'acétate d'éthyle sont ajoutés sous agitation lente. Après quinze minutes, la cristallisation du dipeptide commence. Le produit est maintenu au froid une nuit, filtré sur verre fritté n° 3. Le précipité est lavé par 50 ml d'acétate d'éthyle, puis séché sous vide poussé.

Résultats

Le chlorure de pivaloyle permet d'obtenir le produit attendu avec un bon rendement, et une bonne pureté.

Le dichlorométhane comme solvant permet un enchaînement harmonieux (réaction, extraction, cristallisation). La concentration est de 20 % au départ et 30 % à l'arrivée (la proline étant chargée en poudre). L'acétate d'éthyle permet une véritable cristallisation du produit et une élimination des impuretées formées.

Masse obtenue : 24,3 g

Pf = 147.148° C

$[\alpha]_D$ = 60 (C = 1 MeOH)

Taux de transformation de la Z-Arg $(NO_2)$COOH = 92 %

Rendement sur arginine introduite de la Z-Arg $(NO_2)$ProBzl = 82 %

Dosage de Z-Arg$NO_2$ - Pro Bzl dans le filtrat de cristallisation = 0,3 %.

Des essais supplémentaires ont été réalisés en changeant le réactif d'activation il sont reportés dans le tableau (1).

TABLEAU 1

| N° ESSAI | REACTIF | RAPPORT ProBzl/ Z-ArgNO$_2$ | TEMPS D'ACTIVATION | TT Z-Arg NO$_2$ | RENDEMENT/ Z-Arg NO$_2$ | IMPURETES IDENTIFIEES |
|---|---|---|---|---|---|---|
| 1-2 | 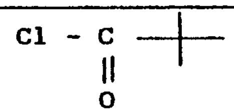 | 1,2 | 2 h à - 5°C<br>40 mn à 0°C | › 95 % | 66,5 % | Lactame/Piv-Pro Bzl* |
| 1-3 | 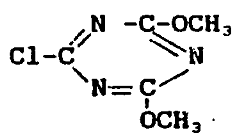 | 1,05 | 3 h à - 5°C | - | 53 % | Lactame-DT-Pro Bzl* |
| 1-4 | 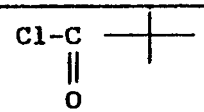 | 1,2 | 1 h 30 à - 5°C | 97,5 % | 87,5 % | Lactame (faible) |
| 1-5 | BOP* | 1 | Température ambiante | - | 72 % | - |
| 1-6 | BOP* | 1 | Température ambiante | - | 85,4 % | - |

* Impuretés :

Lactame 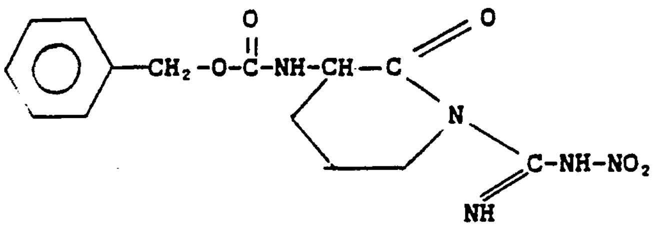

Piv-Pro Bzl : 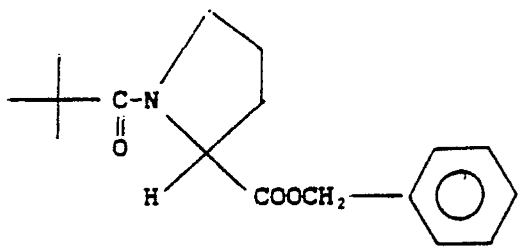

Diméthoxytriazine Pro Bzl : 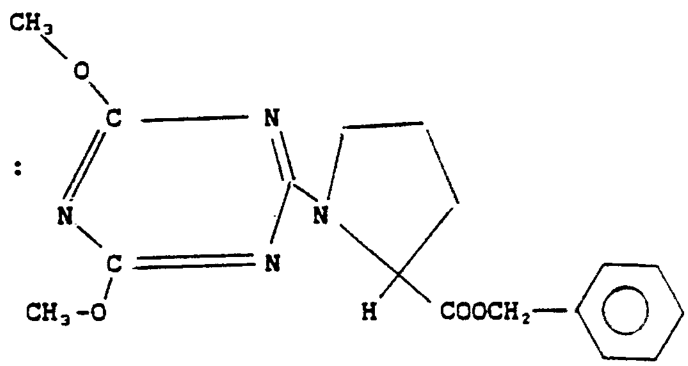

Structure du réactif de Castro (BOP) : 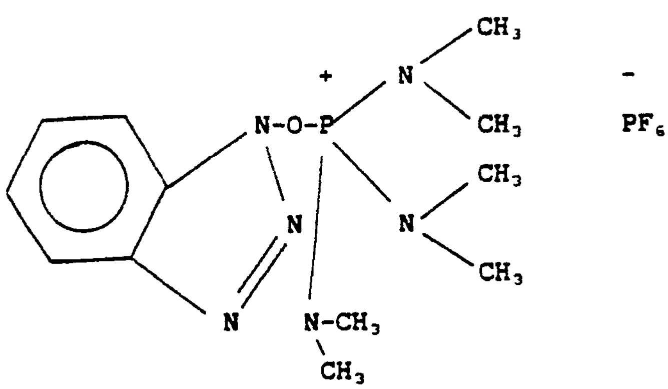

2ème étape du procédé

Mode opératoire

Dans un réacteur tricol de 250 ml, on introduit 21 g de

Z-Arg-Pro-Bzl
$NO_2$

et 80 $cm^3$ d'acide acétique.

La solution est agitée jusqu'à dissolution complète, puis 8ml d'eau, 8 g de palladium à 10 % sur noir de carbone et 3,12 ml de HCl aqueux à 37 % sont ajoutés au milieu réactionnel.

Après purge à l'azote, on fait barbotter l'hydrogène à température ambiante pendant 48 h, sous vive agitation.

Après avoir purgé le réacteur pendant 3 heures, la solution est filtrée sur 2 filtres millipores (LC et LS) WP de porosité (10 microns et 5 microns). Le palladium est rincé par 2 x 25 ml d'eau distillée et la solution est concentrée. On ajoute 100 ml d'eau puis 3,12 ml de HCl aqueux et on concentre sous vide. Le résidu est repris par une fois 100 ml d'eau codistillée puis recodistillé par deux fois avec 100 ml d'éthanol. Le résidu obtenu est repris par 300 ml d'une solution (50/50/v/v/) de méthanol et d'acétate d'éthyle puis est versé sur un litre d'acétate d'éthyle pour obtenir un précipité blanc qui est filtré sur verre fritté n° 3. Le précipité est lavé par 100 ml d'acétate d'éthyle et désséché sous vide poussé sur $P_2O_5$ à température ambiante.

Résultats

Masse obtenue : 12,3 g
Taux de transformation de Z-Arg($NO_2$)Pro-Bzl = 100 %
Rendement en Arg-Pro, 2HCl = 99,3 %

3ème étape

Mode opératoire

Dans un réacteur tricol de 250 ml on charge successivement 5,13 g de Z-L-Leucine, 40 ml de DMF. La solution limpide est agitée puis refroidie à - 15° C. 2,06 ml de N-méthylmorpholine sont ajoutés (réaction légèrement exothermique). Après 5 mn, on ajoute 2,25 ml de chloroformiate d'isobutyle en maintenant la température à - 15° C, la masse réactionnelle blanchâtre est maintenue 10 mn à - 15° C, puis le N-hydroxybenzotriazole est ajouté. Après 20 mn à - 15° C le milieu réactionnel est ramené à 0° C. Après 30 mn à 0° C on coule la solution de Arg-Pro (5,8 g) dans le DMF/$H_2O$(+ $NaHCO_3$) (32 ml/8ml/2,44 g). La réaction est exothermique et la température est maintenue à 0° C pendant 45 mn. On laisse revenir à température ambiante.

Après 2 h 30 de régime, la réaction évolue plus et le DMF est concentré sous vide poussé. Le résidu huileux est repris par 50 ml d'$H_2O$, l'excès de Z-Leucine est éliminé par lavage à l'éther isopropylique. (2 x 25 ml). Cette extraction permet d'éliminer une partie d'hydroxybenzotriazole.

La solution aqueuse est abandonnée au froid pendant une nuit. Une partie du précipité d'hydroxybenzotriazole est éliminée par filtration. Le reste est extrait par plusieurs lavages par le diisopropyléther (5 x 50 ml). Le produit attendu est extrait par un mélange acétate d'éthyle n-BuOH (50/50).

La phase organique est lavée par 2 fois 10 ml de HClN, puis par 2 fois 15 ml d'eau.

La phase organique est concentrée sous vide à 30° C, l'huile visqueuse obtenue est reprise par 50 ml d'isopropanol à chaud (50° C) et versée sur 300 ml d'éther isopropylique pour obtenir un précipité blanc qui est filtré et séché sous vide poussé.

Résultats

Quantité obtenue : 5,5 g
RR isolé = 70 %
Divers essais comparatifs ont été réalisés en faisant varier l'agent d'activation et le solvant.

| N° essai | Rapport AM/Z-Leu Arg-Pro 2HCl | Solvant Activation et température °d'activation | Base Activation | Réactif Activation | Base Neutralisation Arg-Pro, 2HCl | Solvant Dissolution Arg-Pro, 2HCl | Nucléophile auxiliaire | RR Z-Leu Arg-Pro | TT Arg-Pro | HPLC Pureté |
|---|---|---|---|---|---|---|---|---|---|---|
| 3-1 | 1,3 | DMF - 15°C 1 h - 5°C | NMM | 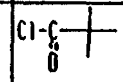 | $NaHCO_3$ 2 éq | $DMF/H_2O$ 90/10 | | 59 % | incomplet | 81 % |
| 3-2 | 1,7 | $CH_2Cl_2$ - 5°C 30 mn - 5°C | NMM | 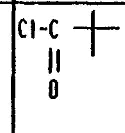 | $NaHCO_3$ 2 éq | $DMF/H_2O$ 80/20 | 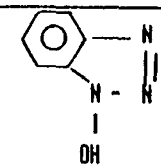 | 27 % | 68 % | |
| 3 -3 | 1,65 | DMF - 5°C | NMM | 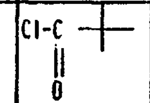 | $NaHCO_3$ 2 éq. | $DMF/H_2O$ 80/20 | 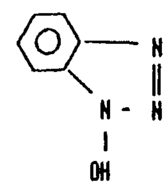 | 70 % | 100 % | |
| 3-4 | 1,16 | DMF - 5°C | NMM | 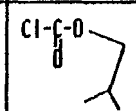 | $NaHCO_3$ 2 éq | $DMF/H_2O$ 80/20 | - | 79 % | 100 % | |
| 3-5 | 1,15 | DMF - 5°C | NMM | 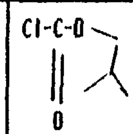 | $NaHCO_3$ 2 éq | $DMF/H_2O$ 80/20 | 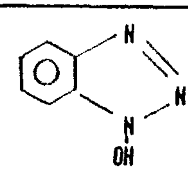 | 89 % (dosé) | 97,5 % | |

| N° essai | Rapport AM/Arg-Pro | Solvant Activation activation | Base Activation | Réactif Activation | Base Neutralisation Arg-Pro, 2HCl | Solvant Dissolution Arg-Pro, 2HCl | Nucléophile auxiliaire ou Autres | RR Z-Leu Arg-Pro | TT Arg-Pro | HPLC Pureté |
|---|---|---|---|---|---|---|---|---|---|---|
| 3-6 | 1,3 | DMF - 15°C | NMM | 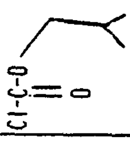 | $NaHCO_3$ 2 éq | DMF/$H_2O$ 80/20 | 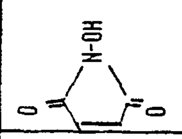 | 67,5 | 75 % | 89,5 % |
| 3-7 | 1,2 | DMF - 15°C | NMM | 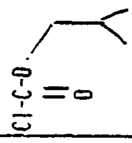 | NMM 2 éq | DMF seul | 2 éq. Cl-$SiMe_3$ Arg-Pro | 77 % | 100 % | 84 % |
| 3-8 | 1,25 | DMF - 15°C 10 mn + OHBT 10 mn - 15°C 20 mn 0°C | NMM | 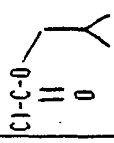 | $NaHCO_3$ 2,55 éq. | DMF/$H_2O$ 80/20 | 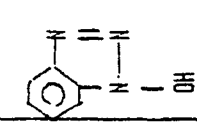 | 85 % | 100 % | 98 % |
| 3-9 | 1,4 | THF - 15°C | NMM | 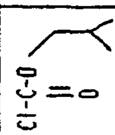 | $NaHCO_3$ 2 éq | DMF/$H_2O$ 90/10 | - | 80 % | non complet | 90 % impuretés |

## Revendications

1. Nouvel intermédiaire de synthèse peptidique caractérisé en ce qu'il répond à la formule L Leucyl - L Arginyl - L Proline

2. Nouvel intermédiaire de synthèse peptidique caractérisé en ce qu'il répond à la formule L Leucyl - L Arginyl - L Proline et, est protégé sur sa fonction amine par un carbamate

3. Procédé de préparation du nouvel intermédiaire selon la revendication 1 caractérisé en ce que
- dans une première étape on met en contact la L nitro Arginine, protégée sur sa fonction amine, avec un activateur puis avec la L Proline protégée sur sa fonction acide en présence d'au moins une base dans un

solvant organique,
- dans une deuxième étape on réalise une hydrogénation en milieu acide du dipeptide obtenu à la première étape,
- dans une troisième étape on active la L leucine protégée sur sa fonction amine puis on la met en contact après addition d'un nucléophile auxiliaire avec le dipeptide obtenu à la deuxième étape en présence d'une base.

4. Procédé selon la revendication 3 caractérisé en ce que le groupe protecteur de la nitroarginine est le groupe benzyloxycarbonyle et celui de la proline est le groupe benzyle.

5. Procédé selon la revendication 3 caractérisé en ce que l'activateur de la première étape est le chlorure de pivaloyle.

6. Procédé selon la revendication 3 caractérisé en ce que lors de la mise en contact de la L nitroarginine avec un activateur on ajoute avantageusement une base faible.

7. Procédé selon la revendication 6 caractérisé en ce que la base faible est la Nméthylmorpholine.

8. Procédé selon la revendication 3 caractérisé en ce que lors de la mise en contact de la L nitroarginine protégée et activée avec la L Proline protégée on ajoute une base choisie parmi la triethylamine et le carbonate acide de sodium.

9. Procédé selon la revendication 3 caractérisé en ce que le dipeptide de Arginine - Proline protégé est cristallisé dans l'acètate d'éthyle

10. Procédé selon la revendication 3 caractérisé en ce que lors de la 2ème étape on ajoute un catalyseur d'hydrogénation à base d'un métal précieux déposé sur un support.

11. Procédé selon la revendication 10 caractérisé en ce que le métal précieux est le palladium.

12. Procédé selon la revendication 10 caractérisé en ce que le support est choisi parmi le charbon, la silice, l'alumine et le sulfate de baryum.

13. Procédé selon les revendications 11 et 12 caractérisé en ce que le catalyseur est à base de palladium déposé sur charbon.

14. Procédé selon les revendications 10 et 13 caractérisé en ce que le rapport pondéral du métal précieux au dipeptide est compris entre 0,5 et 5 %.

15. Procédé selon la revendication 3 caractérisé en ce que l'acide utilisé lors de la deuxième étape est l'acide chlorhydrique.

16. Procédé selon la revendication 3 caractérisé en ce que l'activateur utilisé lors de la 3ème étape est choisi parmi le chloroformiate d'isobutyle et le chlorure de pivaloyle.

17. Procédé selon les revendications 3 et 16 caractérisé en ce que l'on ajoute en même temps que l'activateur une base qui est la N méthylmorpholine.

18. Procédé selon la revendication 3 caractérisé en ce que le nucléophile auxiliaire est choisi parmi l'hydroxybenzotriazole, l'hydroxysuccinimide et le thiazolidine-thiol.

19. Procédé selon la revendication 18 caractérisé en ce que le nucléophile auxiliaire est l'hydroxybenzotriazole.

20. Procédé selon la revendication 3 caractérisé en ce que lors de la mise en contact de la L leucine activée et du dipeptide obtenu à la 2ème étape on ajoute une base choisie parmi la triethylamine et le carbonate acide de sodium.

Office européen des brevets

# RAPPORT DE RECHERCHE EUROPEENNE

Numero de la demande

EP 90 40 1338

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | EP-A-0 033 976 (TAKEDA)<br>* Page 0, lignes 11-12 *<br>--- | 1-4,8-9,10 | C 07 K 5/08 |
| X | FR-A-2 329 294 (D. PARKE)<br>* Pages 0-9; page 18, ligne 33 - page 19, ligne 20 *<br>--- | 1-4,8-9,10 | |
| X,Y | DE-A-2 347 151 (AMER. HOME PR)<br>* Pages 0,17,26-27 *<br>--- | 1-4,8-9,10 | |
| Y | FR-A-2 277 078 (TAKEDA)<br>* Pages 0,19-20,25-26 *<br>----- | 1,8-9,10 | |

DOMAINES TECHNIQUES RECHERCHES (Int. Cl.5)

C 07 K

Le présent rapport a été établi pour toutes les revendications

| Lieu de la recherche | Date d'achèvement de la recherche | Examinateur |
|---|---|---|
| LA HAYE | 30-08-1990 | RAJIC M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant